# EUROPEAN PATENT APPLICATION

(11) **EP 1 975 619 A1**
(43) Date of publication of application: **01.10.2008**
(21) Application number: 08005577.5
(22) Date of filing: 26.03.2008
(51) Int. Cl.: G01N 33/558

(54) **Chromatographic test device and method for detecting analyte**

(30) Priority: 30.03.2007 JP 2007093569
(71) Applicant: SYSMEX CORPORATION, Hyogo 651-0073 (JP)
(72) Inventor: Horisaka, Kanako c/o Sysmex Corporation, Hyogo 673-0036 (JP); Manabe, Tomoko c/o Sysmex Corporation, Chuo-ku, Kobe-shi Hyogo 651-0073 (JP)
(74) Representative: HOFFMANN EITLE

(57) **Abstract**

The present invention provides a chromatographic test device, comprising a firstbloodcell separationmember, a second blood cell separation member and a chromatographic carrier disposed in this order from the upstream side of the direction of development of a sample, wherein the first blood cell separation member is constituted so as to separate the blood cell components and the liquid components from each other based on a development speed; the second blood cell separation member is constituted so as to separate the blood cell components and the liquid components from each other based on filtration; and the chromatographic carrier carries a capturing substance being capable of binding specifically to the analyte. A method for detecting the analyte in the sample containing red blood cells and liquid components by using the chromatographic test device is also disclosed.

## Description

### FIELD OF THE INVENTION

The present invention relates to a chromatographic test device and a method for detecting an analyte.

### BACKGROUND

As a test device for detecting an analyte in a sample by using a substance binding specifically to the analyte, there is a chromatographic test device. It is known that when an analyte in blood is detected with a chromatographic test device, accurate reading of its detection result is made difficult due to a color derived from red blood cells in the blood. It follows that when a sample containing blood cells such as red blood cells is used, a chromatographic test deviceprovidedwitha separation membrane for separating blood cells (particularly red blood cells) from the sample is used.

Known chromatographic test devices provided with a separation membrane for separating blood cells are as follows:
US6008059 describes a chromatographic test device provided with a porous separation matrix for removing cell components such as red blood cells. It is also described therein that as the porous separation matrix, an asymmetric membrane constituted so as to have a gradient of decreasing pore size in the membrane can be used.
US2003032196 describes a chromatographic test device provided with a sample filter for removing cell components such as red blood cells.
W09734148 describes a chromatographic test device provided with a porous membrane for separation based on a higher development speed of liquid components than that of cell components such as red blood cells. It is described therein that in this porous membrane, red blood cells are not filtered out from whole blood, but are separated from plasma because of a lower development speed of red blood cells.

The chromatographic test devices in US6008059 and US2003032196 supra make use of a separation membrane that captures blood cell components such as red blood cells in a sample and simultaneously allows liquid components to pass, thereby separating the blood cell components. When a blood sample is added to a separation membrane in such a chromatographic test device, blood cell components in the blood sample are captured by the separation membrane. At this time, the separation membrane may be clogged to take a longer time in allowing liquid components (for example, plasma etc.) in blood to pass through the separation membrane. In this case, there is a problem that the examination is time-consuming.

The chromatographic test device in WO9734148 supra makes use of a separation membrane in which blood cell components are separated based on a higher development speed of liquid components in a sample than that of blood cell components such as red blood cells. In such a chromatographic test device, blood cell components are not captured in the separation membrane. Accordingly, red blood cells etc. will be leaked from the separation member with time after examination, to erode a chromatographic carrier. Accordingly, there is a problem that reading of examination results is made difficult.

### SUMMARY

The scope of the present invention is defined solely by the appended claims, and is not affected to any degree by the statements within this summary.

### BRIEF DESCRIPTION OF THE DRAWINGS

FIG. 1 is a cross-sectional view showing the structure of a chromatographic test device in one embodiment of the invention;
FIG. 2 is a cross-sectional view showing a modified example of the chromatographic test device in FIG. 1;
FIG. 3 is a cross-sectional view showing the structure of the chromatographic test device of the invention in Example 1;
FIG. 4 is a cross-sectional view showing the structure of a chromatographic test device in Comparative Example 1;
FIG. 5 is a cross-sectional view showing the structure of the chromatographic test device of the invention in Example 2;
FIG. 6 is a cross-sectional view showing the structure of a chromatographic test device in Comparative Example 2;
FIG. 7 is a photograph showing a developed state with time of a sample added to the chromatographic test devices in Example 1 and Comparative Example 1 of the invention.
FIG. 8 is a photograph showing a developed state with time of a sample added to the chromatographic test devices in Example 2 and Comparative Example 2 of the invention.

### DETAILED DESCRIPTION OF THE EMBODIMENT

Hereinafter, embodiments of the present invention are described in detail by reference to the drawings. The embodiments shown in the drawings and in the following description are provided for illustrative purposes only, and the scope of the present invention should not be construed as being limited to the embodiments shown in the drawings and in the following description.

### 1. Constitution of the chromatographic test device

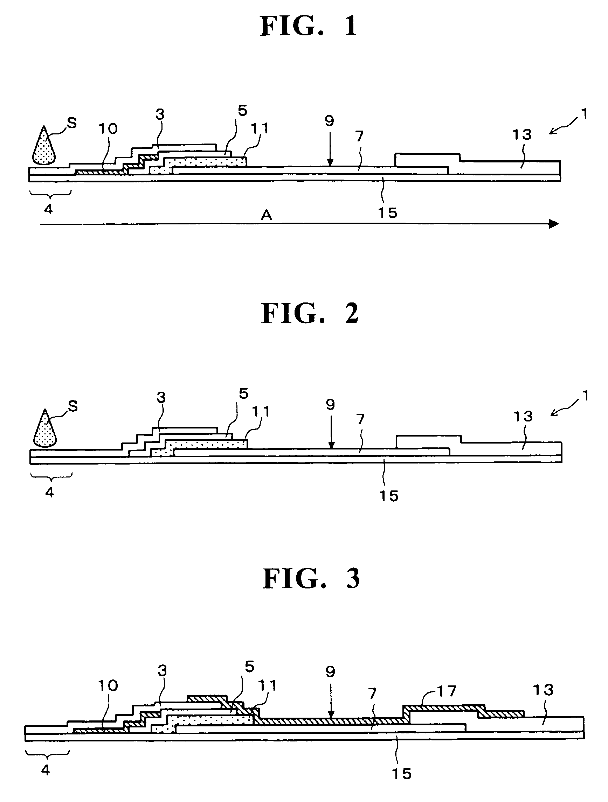

FIG. 1 is a cross-sectional view showing the structure of a chromatographic test device (also referred to hereinafter as "test device") in one embodiment of the invention. As shown in FIG. 1, a test device 1 is provided with a first blood cell separation member 3, a second blood cell separation member 5 and a chromatographic carrier 7 in the order of upstream to downstream of the direction of development (direction indicated by an arrow A) of a sample containing blood cell components and liquid components. The first blood cell separation member 3 is constituted so as to separate the blood cell components and the liquid components from each other based on a higher development speed of the liquid components than that of blood cell components in the first blood cell separation member 3. The second blood cell separation member 5 is constituted so as to separate the blood cell components and the liquid components from each other based on capture of the blood cell components and simultaneous passage of the liquid components.

The chromatographic carrier 7 carries, on a judgment part 9, a capturing substance being capable of binding specifically to an analyte. The first blood cell separation member 3 is constituted so as to separate a sample S in the direction of sample development. The second blood cell separation member 5 is constituted so as to separate the sample S in a direction perpendicular to the direction of sample development. The first blood cell separationmember 3 overlaps partially with the second blood cell separation member 5. The first blood cell separation member 3 has a sample addition part 4 in its region not overlapping with the second blood cell separation member 5.

Generally, a chromatographic test device is constituted such that when a sample is added to a predetermined part (for example, the sample addition part 4 in FIG. 1), the sample is developed (transferred) toward a part where its detection result is indicated (for example, the judgment part 9 in FIG. 1). As used herein, "the direction of sample development" refers to the direction inwhich a sample is developed (transferred) toward the part where its detection result is indicated.

A sealing member 10 for preventing the infiltration of the sample S from the first blood cell separation member 3 to the second blood cell separation member 5 is disposed on the edge of the second blood cell separation member 5 on the upstream side of the direction of sample development.

The second blood cell separation member 5 and a chromatographic carrier 7 are provided therebetween with a label holding member 11. The label holding member 11 is disposed in contact with the second blood cell separation member 5. The label holding member 11 is disposed in contact with the chromatographic carrier 7. The label holding member 11 has a labeling substance being capable of binding specifically to an analyte and being labeled.

An absorbent member 13 is disposed on the downstream side of the chromatographic carrier 7, so as to contact with the chromatographic carrier 7. Each of the members is attached to a base material 15 consisting of plastics, paper or glass. 2. Method of using the chromatographic test device, etc.

Hereinafter, a method of using the chromatographic test device 1 is described by reference to FIG. 1. It will also be described that when the test device 1 is used, rapid examination is feasible, and even if time passes after examination, the chromatographic carrier 7 is hardly eroded with blood cell components such as red blood cells.

The direction in which the test device 1 is disposed at the time of examination is not particularly limited. The test device 1 may be used in a horizontally disposed state or may be used in a vertically disposed state. Hereinafter, the method is described by reference to the case where the test device 1 is used in a horizontally disposed state.

### (1) Addition of sample S

First, a sample S containing blood cell components and liquid components is added to the sample addition part 4 in the first blood cell separation member 3. At this point, it is not evident whether an intended analyte is contained in the sample S is contained or not. In the following description, it is assumed that the analyte is contained in the sample.

As long as the sample S contains blood cell components and liquid components, the sample may be blood itself, a dilution of blood in a developing solvent or the like, or blood pretreated (for example, to remove a part of the blood components). The blood cell components comprise blood cells such as red blood cells. When the sample S is blood itself, the liquid components comprise plasma in blood. When the sample S is blood to which a developing solvent was added, the liquid components are composed of plasma and the developing solvent. The sample addition part 4 is the part of the test device to which the sample is added. The sample addition part 4 refers to a site that is assigned, by manufacture's instructions or by an indication on the test device 1 itself or on a container accommodating the test device 1, as the site to which the sample is to be added. The sample addition part 4 is disposed preferably in a position as far away from the second blood cell separation member 5 as possible. Particularly, the sample addition part 4 is disposed on the edge, in the upstream side, of the test device 1. For example, the sample addition part 4 is disposed on the region of the first blood cell separation 3 that does not overlap with the second blood cell separation member 5. In this way, the sample addition part 4 is disposed in a position as far away from the second blood cell separation member 5 as possible, thereby increasing the distance at which the sample S migrates in the first blood cell separation member 3. As a result, the blood cell components and the liquid components that are contained in the sample S are suitably separated from each other. When the separation length is too long relative to the amount of the sample, there is a possibility that the liquid components in an amount enough to detect an analyte cannot reach the second blood cell separation member. Accordingly, the separation length is suitably determined depending on the amount of the sample.

The sample S added to the sample addition part 4 permeates into the first blood cell separation member 3 and migrates, by capillary phenomenon, in the first blood cell separation member 3, the second blood cell separation member 5, the label holding member 11, the chromatographic carrier 7 and the absorbent member 15 in this order. Accordingly, these members can make use of materials (for example, nonwoven fabrics, porous members etc.) capable of developing the sample S by capillary phenomenon.

### (2) Separation with the first blood cell separation member

Liquid components and blood cell components in the sample S are separated from each other due to a difference in development speed therebetween in the first blood cell separation member 3. That is, the liquid components in the sample S are initially discharged from the first blood cell separation member 3. The blood cell components in the sample S are discharged later than the liquid components, from the first blood cell separation member 3. A certain amount of the blood cell components can be contained in the initially discharged liquid components, but does not significantly influence the working effect of this embodiment. In the following description, therefore, it is assumed that the blood cell components are substantially not contained in the initially discharged liquid components.

The constitution of the first blood cell separation member 3 is not limited insofar as the first blood cell separation member 3 is constituted so as to separate the blood cell components and the liquid components from each other based on a higher development speed, in the first blood cell separation member 3 , of the liquid components than that of the blood cell components such as red blood cells. The action of the first blood cell separation member 3 by which the developing speed of the liquid components is made higher than that of the blood cell components can be exemplified by certain interactions between the blood cell components and the first blood cell separation member 3 (for example, electrostatic interaction, steric interaction) by which the development speed of the blood cell components in the first blood cell separation member 3 is reduced. In one example, if the first blood cell separation member 3 consists of a porous member and its pores is equal in size to or slightly larger than blood cells, then the liquid components can move smoothly in the pores, while the blood cell components cannot move smoothly in the pores, and thus the development speed of the blood cell components in the first blood cell separation member 3 is reduced and the development speed of the liquid components is made higher than that of the blood cell components.

Blood cells contained in blood vary in size depending on their type. Therefore, the sample S can contain blood cell components of various sizes. For example, when the sample S is prepared from human blood, the blood cells that can be contained in the sample S and their sizes are as follows: Among leukocytes, lymphocytes are 6 to 15 µm in size, monocytes are 12 to 18 µm, neutrophils are 10 to 12 µm, basophils are 10 to 15 µm and eosinophils are 10 to 15 µm, and red blood cells are 7 to 8 µm and platelets are 1 to 4 µm. Blood cell components of relatively small size such as platelets that can be contained in the sample S may have a higher development speed in the first blood cell separation member 3 than that of blood cell components of relatively large size such as red blood cells and may, depending on the case, have approximately the same development speed as that of the liquid components. However, blood cell components of relatively smaller size than blood cell components such as red blood cells are unlikely to exert an influence on detection results, and thus do not significantly influence the working effect in this embodiment even if their development speed in the first blood cell separation member 3 is increased.

The first blood cell separation member 3 can make use of a membrane used in paper chromatography or thin-layer chromatography. Examples of such usable membrane are membranes made of various materials such as nitrocellulose, nylon (for example, modified nylon into which a carboxyl group, and an amino group which may be substituted with an alkyl group, have been introduced), polyvinylidene difluoride (PVDF) and cellulose acetate. Specifically, commercial products such as MF1 (lateral flow type, Whatman), LF1 (lateral flow type, Whatman) and FUSION5 (Whatman), or members described in W09734148, may be used.

The first blood cell separation member 3 is preferably membranous. The first blood cell separation member 3 is preferably that which transfers the sample S in the direction of sample development, to separate the sample S in the direction of sample development. This is because the distance (separation distance) at which the sample S migrates in the first blood cell separation member 3 can be easily made longer. However, the constitution of the first blood cell separation member 3, and the direction of sample separation with the first blood cell separation member 3, are not particularly limited. For example, the first blood cell separation member 3 may be that which is in the form of a block or separates the sample S in a direction perpendicular to the direction of sample development.

The liquid components discharged from the first blood cell separation member 3 pass through the area of contact of the first blood cell separation member 3 with the second blood cell separation member 5 and migrate to the second blood cell separation member 5. In this embodiment, the first blood cell separation member 3 and the second blood cell separation member 5 are contacted directly with each other, but the first blood cell separation member 3 and the second blood cell separation member 5 may be provided therebetween with another member not preventing the sample S from migrating from the first blood cell separation member 3 to the second blood cell separation member 5 . The sample S can penetrate, either in the direction of sample development or in a direction perpendicular to the direction of sample development, from the first blood cell separation member 3 to the second blood cell separation member 5. In this embodiment, the second blood cell separation member 5 is constituted so as to separate the sample S in a direction perpendicular to the direction of sample development, so it is preferable that the sample S penetrates, in a direction perpendicular to the direction of sample development, into the second blood cell separation member 5. Accordingly, a sealing member 10 for preventing the infiltration of the sample S from the first blood cell separation member 3 to the second blood cell separation member 5 is disposed on the edge of the second blood cell separation member 5 at the upstream side of the direction of sample development. The sample S can thereby be prevented from penetrating into the secondblood cell separation member 5 in the direction of sample development. As long as the sealing member 10 can prevent penetration of the sample S, its constitution and material are not particularly limited. Specifically, commercial products such as ARcare series (Adhesives Corporation) may be used as the sealing member. As shown in FIG. 2, the sealing member 10 may be omitted.

### (3) Separation with the second blood cell separation member

The liquid components transferred to the second blood cell separation member 5 move in the second blood cell separation member 5, in a direction perpendicular to the direction of sample development. The second blood cell separation member 5 is that which separates the liquid components and blood cell components in the sample S from each other by capturing the blood cell components and allowing the liquid components to pass. That is, the blood cell components such as red blood cells are captured by the second blood cell separation member 5, while the liquid components without being captured are discharged from the second blood cell separation member 5. In this embodiment, the liquid components first pass through the second blood cell separation member 5. It follows that before the passage of the liquid components, the second blood cell separation member 5 is not clogged with the blood cell components, and therefore, the liquid components pass smoothly through the secondblood cell separation member 5. In this embodiment, therefore, examination can be rapidly carried out.

The blood cell components in the sample S are discharged, later than the liquid components, from the first blood cell separation member 3 and reach the second blood cell separation member 5. Because the second blood cell separation 5 captures the blood cell components such as red blood cells thereby preventing the blood cell components from passing therethrough, the chromatographic carrier 7 will, even after passage of time, not be eroded with the blood cell components such as red blood cells. Accordingly, reading of examination results is not made difficult even after passage of time. At this time, the second blood cell separation member 5 can be clogged, but the liquid components have already passed through the second blood cell separation member 5, and thus the examination time is not influenced.

The constitution of the secondblood cell separationmember 5 is not limited as long as the second blood cell separation member 5 is constituted so as to separate the blood cell components such as red blood cells and the liquid components from each other based on capture of at least red blood cells and simultaneous passage of the liquid components. By way of example, the second blood cell separation member 5 consists of a porous member having pores smaller than blood cells (for example, red blood cells) to be captured. In this case, blood cells to be captured cannot pass through the pores and are captured by the second blood cell separation member 5. The size of pores in the porous member may be decreased in the direction of from the upper to lower surfaces of the porous member. In this case, pores at the side of the upper surface of the porous member are relatively large to allow the sample to spread easily, and the sample arrives, in a sufficiently spread state, at pores of relatively small size at the side of the lower surface of the porous member, and the blood cell components are captured by these pores, so there is an advantage that efficient separation is feasible.

As described above, blood cell components of various sizes can be contained in the sample S. Blood cell components of relatively smaller size than any of the blood cell components such as red blood cells contained in the sample S may, without being captured in the second blood cell separation member 5, pass therethrough together with the liquid components. However, blood cell components of relatively smaller size than any of the blood cell components such as red blood cells are unlikely to exert an influence on detection results. Accordingly, even if the blood cell components of relatively small size have passed together with the liquid components through the second blood cell separation member 5, they do not significantly influence the working effect in this embodiment. Accordingly, the same effect as when "the blood cell components are captured" can be attained in this case too.

The size of blood cells such as red blood cells varies depending on the animal species from which they are derived. For example, canine red blood cells and feline red blood are respectively about 80% and about 50% in size relative to human red blood cells. It follows that, for example, when red blood cells are to be captured, the size of pores in the second blood cell separation member 5 is suitably selected depending on the size of red blood cells of the animal species from which blood used as a sample is derived.

The second blood cell separation member 5 can make use of a commercially available filtration membrane. The commercially available filtration membrane includes, for example, BTS SP 100, BTS SP 200 and BTS SP 300 (Vertical Flow Type, PALL Corporation). The second blood cell separation member 5 can also make use of an asymmetric membrane described in US6008059 and a sample filter in US2003032196. For example, the asymmetric membrane or the sample filter can be produced using materials prepared from a combination of a hydrophobic polymer and a hydrophilic polymer (US5240862, US5076925). The hydrophobic polymer includes polysulfone, polyether sulfone, polyimide and polyether imide. The hydrophilic polymer includes polyvinyl pyrrolidone, polyacrylic acid, polyvinyl alcohol, polyvinyl acetate and polyethylene glycol.

The second blood cell separation member 5 is preferably membranous. The second blood cell separation member 5 is preferably that which transfers the sample S in a direction perpendicular to the direction of sample development, to separate the sample S in a direction perpendicular to the direction of sample development. When liquid components and blood cell components are separated from each other by capturing the blood cell components and allowing passage of the liquid components, the separation area is generally preferably larger. The separation area of the second blood cell separation member 5 that separates the sample S in a direction perpendicular to the direction of sample development can be easily increased. However, the constitution of the second blood cell separation membrane 5, and the direction of sample separation with the second blood cell separation member 5, are not particularly limited. For example, the second blood cell separation member 5 may be in the form of a block, or it may be that which separates the sample S in the direction of sample development. When the separation area is too large relative to the amount of the sample, there is a possibility that the liquid components in an amount enough to detect an analyte cannot reach the chromatographic carrier 7. Accordingly, the separation area is suitably determined depending on the amount of the sample S.

When the second blood cell separation member 5 separates the sample S in a direction perpendicular to the direction of sample development, at least a part of the first blood cell separation member 3 is disposed preferably so as to overlap, on the upstream side of the direction of sample development in the second blood cell separation member 5, with the second blood cell separation member 5. At least a part of the second blood cell separation member 5 is disposed preferably so as to overlap, on the upstream side of the direction of sample development in the chromatographic member 7, with the chromatographic member 7. By such arrangement, the sample S discharged from the first blood cell separation member 3 can be received in a broader area at the side of the upper surface of the secondblood cell separation member 5. Further, the sample S can be discharged to a broader area of the chromatographic carrier 7 at the side of the lower surface of the second blood cell separation member 5.

When the end of the second blood cell separation member 5 on the downstream side of the direction of sample development overlaps with the first blood cell separation member 3, the sample S discharged from the first blood cell separation member 3 may, without passing through the second blood cell separation member 5, reach the chromatographic carrier 7. Accordingly, the second blood cell separation member 5 is preferably provided with a region not overlapping with the first blood cell separation member 3 on the downstream side of the direction of sample development.

The liquid components discharged from the second blood cell separation member 5 pass through the area of contact of the second blood cell separation member 5 with the label holding member 11 and migrate to the label holding member 11. In this embodiment, the second blood cell separation member 5 and the label holding member 11 are contacted directly with each other, but the secondblood cell separationmember 5 and the label holding member 11 may be provided therebetween with another member not preventing the sample S from migrating from the second blood cell separation member 5 to the label holding member 11.

### (4) Formation of a complex

The liquid components transferred to the label holding member 11 moves in the label holding member 11 by capillary phenomenon. The label holding member 11 has a labeling substance being capable of binding specifically to an analyte and being labeled. Accordingly, when an analyte is contained in the liquid components, the labeling substance binds specifically to the analyte to form a complex. The formed complex together with the liquid components moves to the chromatographic carrier 7. An analyte and the labeling substance, which are in a form not forming the complex (that is, in a separated state), also move together with the liquid components to the chromatographic carrier 7.

As long as the analyte (the substance to be detected) is the one contained in blood such as human blood and not filtrated with the second blood cell separation member 5, its type is not particularly limited. Examples of the analyte include antigens, antibodies, hormones, hormone receptors, lectins, lectin-binding carbohydrates, drugs or metabolites thereof, drug receptors, nucleic acids, and fragments, variants and combinations thereof. Other examples of the analyte include cells such as bacteria cells, protist cells and mycete cells, viruses, proteins and polysaccharides, which can be exemplified by influenza viruses, parainfluenza virus, RS virus, *Mycoplasma pneumoniae,* rotavirus, calicivirus, coronavirus, adenovirus, enterovirus, herpes virus; human immunodeficiency virus, hepatitis virus, pathogenic viruses causing sever acute respiratory syndrome, *Escherichia coli, Staphylococcus aureus, Streptococcus pneumoniae, Streptococcus pyogenes,* malaria parasite, pathogens causing various diseases such as digestive system disease, central nervous system diseases and hemorrhagic fever, metabolites thereof, tumor markers such as carcinoembryonic antigen and CYFRA, and hormones.

When canine blood is used, the analyte is further exemplified by filarial worm antigens. When feline blood is used, the analyte is further exemplified by feline leukemia virus, feline immunodeficiency virus and antibodies thereof.

The labeling substancemaybe thatwhichbinds specifically to the analyte. The specific bond between the analyte and the labeling substance includes bonds based on biological affinity, such as a bond due to an antigen-antibody reaction and a ligand-receptor bond. The labeling substance can be labeled by various methods. The labeling substance can be labeled, for example, with colored particles. In this case, examination results can be confirmed with the naked eye. The colored particles include, for example, colored latex particles, metallic colloids such as gold colloids, etc.

The label holding member 11 can make use of various materials such as glass fiber and cellulose fiber. The label holding member 11 may be omitted. In this case, the labeling substance has previously been added to the sample S or held in other members such as first or secondblood cell separationmember 5, 5, etc.

The liquid components discharged from the label holding member 11 pass through the area of contact of the label holding member 11 with the chromatographic carrier 7 and migrate to the chromatographic carrier 7. In this embodiment, the label holding member 11 and the chromatographic carrier 7 are contacted directly with each other, but the label holding member 11 and the chromatographic carrier 7 may be provided therebetween with another member not preventing the sample S from migrating from the label holding member 11 to the chromatographic carrier 7.

### (5) Judgment of examination results

The liquid components transferred to the chromatographic carrier 7 moves in the chromatographic carrier 7 by capillary phenomenon. The chromatographic carrier 7 carries, in the judgment part 9, the labeling substance being capable of binding specifically to an analyte. Accordingly, when the liquid components reach the judgment part 9, the analyte in the liquid components is bound specifically to the labeling substance. As a result, the analyte is captured in the judgment part 9. When the labeling substance is bound to the analyte captured in the judgment part 9, a label with which the labeling substance is labeled appears on the judgment part 9. For example, if the labeling substance is labeled with colored particles, the color of the particles will appear on the judgment part 9, and it is thus confirmed that the analyte is present in the sample S. There can be both the case where after the analyte is bound to the labeling substance, the analyte is bound to the capturing substance and the case where after the analyte is bound to the capturing substance, the analyte is bound to the labeling substance.

The capturing substance may be that which binds specifically to an analyte. The specific bond between the capturing substance and the analyte includes, for example, bonds based on biological affinity, such as a bond due to an antigen-antibody reaction and a ligand-receptor bond.

For example, when the analyte is an antigen, antibodies against the antigen can be used as the labeling substance and the capturing substance. In this case, an antibody used as the labeling substance and an antibody used as the capturing substance are preferably those recognizing different sites (epitopes) of the antigen respectively.

For example, when the analyte is an antibody, an antigen against the antibody can be used as the capturing substance, and a substance (antibody) binding specifically to the antibody can be used as the labeling substance.

The liquid components that have passed through the judgment part 9 are discharged from the chromatographic carrier 7 and absorbed into the absorbent member 13. The absorbent member 13 absorbs the liquid components thereby promoting the movement of the liquid components. The absorbent member 13 can also be omitted. In this case, the movement of the liquid components may be promoted, for example, by making the chromatographic carrier 7 longer.

The chromatographic carrier 7 may be prepared from a variety of materials such as nitrocellulose, nylon (for example, modified nylon into which a carboxyl group, and an amino group which may be substituted with an alkyl group, have been introduced), polyvinylidene difluoride (PVDF) and cellulose acetate. The absorbent member 13 may be prepared from a variety of materials such as cellulose and glass fiber.

Depending on the situation under which examination is conducted, there is the case where the sample S is added to the sample addition part 4 in the test device 1, then the test device 1 is left as it is, and after passage of time, its examination result is confirmed. In such case, the blood cell components discharged later than the liquid components from the first blood cell separation member may reach the judgment part 9 and influence the judgment result of the judgment part 9. For example, when red blood cells reach the judgment part 9, the judgment part 9 will be stained red thus making it difficult to recognize a color appearing on the judgment part 9 as the result of detection of the analyte. In this embodiment, however, the blood cell components discharged from the first blood cell separationmember are captured by the second blood cell separation member 5, and thus the blood cell components are prevented from reaching the judgment part 9 so that even left after examination, the judgment part 9 will not undergo the influence of the blood cell components.

### EXAMPLE

### 1. Preparation of the test device in Example 1

As shown in FIG. 3, the test device in Example 1 was prepared. Specifications for members are as shown in Table 1. Overlapping among the members is as shown in FIG. 3. In the test device in Example 1, the overlapping between a protective sheet 17 and a first blood cell separation member 3 is so small that the development speed of a sample added is relatively high.

**Table 1**

| Constitution of the test device in Example 1 | | | |
|---|---|---|---|
| Name | Size | Type | Manufacture |
| First blood cell separation member 3 | 30 mm×5 mm | MF1 | Whatman |
| Separation blood cell separation member 5 | 5 mm×5 mm | BTS SP300 | PALL |
| Sealing member 10 | 5 mmx5 mm | 7759 | Adhesives |
| Label holding member 11 | 5 mmx5 mm | 8975 | PALL |
| Chromatographic carrier 7 | 20 mm×5 mm | SHF 13504 | Millipore |
| Base material 15 | 85 mm | 9020 | Adhesives |
| Protective sheet 17 | 32 mm | 7759 | Adhesives |
| Absorbent member 13 | 40 mmx5 mm | CF5 | Whatman |

### 2. Preparation of the test device in Comparative Example 1

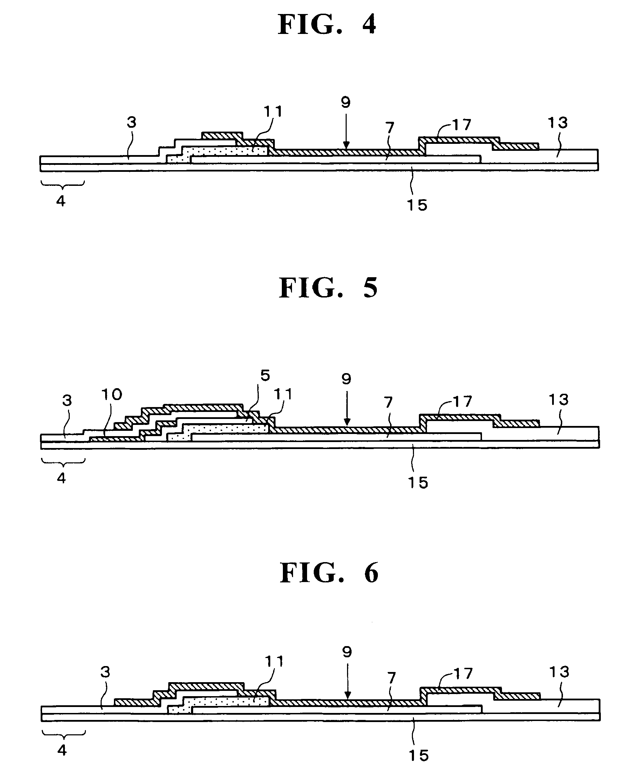

Then, the test device in Comparative Example 1 was prepared as shown in FIG. 4. The test device 1 in Comparative Example 1 has the same constitution as that of the test device in Example 1 except that the test device is not provided with the second blood cell separation member 5 and the sealing member 10. Accordingly, specifications for the members are as shown in Table

### 2. Overlapping among the members are as shown in FIG. 4.

**Table 2**

| Constitution of the test device in Comparative Example 1 | | | |
|---|---|---|---|
| Name | Size | Type | Manufacture |
| First blood cell separation member 3 | 30 mm×5 mm | MF1 | Whatman |
| Label holding member 11 | 5 mm×5 mm | 8975 | PALL |
| Chromatographic carrier 7 | 20 mm×5 mm | SHF 13504 | Millipore |
| Base material 15 | 85 mm | 9020 | Adhesives |
| Protective sheet 17 | 32 mm | 7759 | Adhesives |
| Absorbent member 13 | 40 mm×5 mm | CF5 | Whatman |

### 3. Preparation of the test device in Example 2

The test device in Example 2 was prepared as shown in FIG. 5. The test device in Example 2 is similar to the test device in Example 1, but is different from the test device in Example 1 in that the protective sheet 17 is longer and the overlapping between the protective sheet 17 and the first blood cell separation member 3 is larger. Accordingly, the development speed of a sample added is relatively low in the test device in Example 2.

### 4. Preparation of the test device in Comparative Example 2

The test device in Comparative Example 2 was prepared as shown in FIG. 6. The test device in Comparative Example 2 is similar to the test device in Comparative Example 1, but is different from the test device in Comparative Example 1 in that the protective sheet 17 is longer and the overlapping between the protective sheet 17 and the first blood cell separationmember 3 is larger. In addition, the test deice in Comparative Example 2 has the constitution in which the second blood cell separation member 5 and the sealing member 10 are removed from the test device in Example 2.

### 5. Test for confirmation of effect

Then, a test for demonstrating the effect of the present invention was carried out by the following method.

### 5-1. Comparison between the test device in Example 1 and the test device in Comparative Example 1

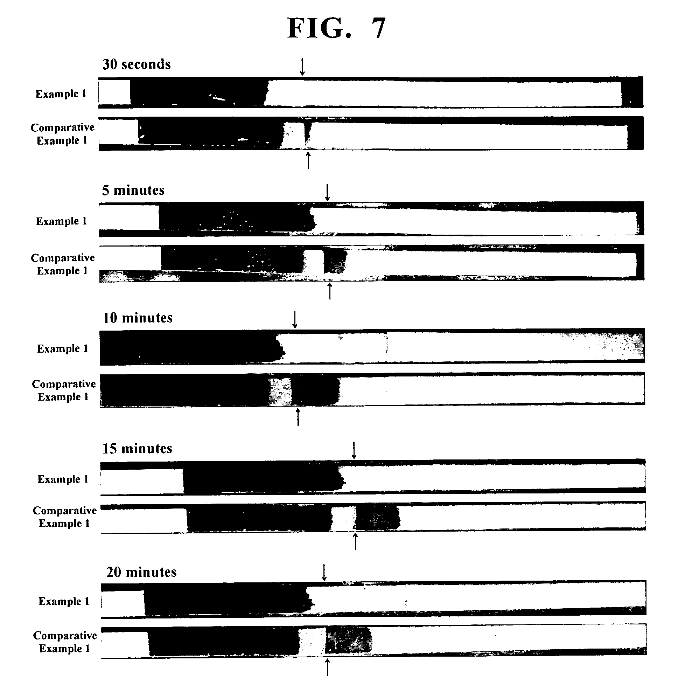

The length of leakage with time of blood cells was observed and measured in the case where 100 µL of whole blood was added to, and developed in, each of the test device in Example 1 and the test device in Comparative Example 1. The results are shown in Table 3 and FIG. 7. FIG. 7 is a photograph showing the developed states of the sample with time in the test devices in Example 1 and Comparative Example 1. In the photograph in FIG. 7, the end, on the downstream side of the direction of sample development, of the label holding member 11 is shown by an arrow. The length of a part on the right side relative to this arrow was regarded as the length of leakage of blood cells. The ratio of the length of leakage of blood cells to the length of that part of the chromatographic carrier 7 that overlaps with neither the label holding member 11 nor the absorbent member 13 was regarded as occupancy and shown in Table 3.

**Table 3**

| | Test device in Example 1 | Test device in Comparative Example 1 |
|---|---|---|
| Elapsed time | Occupancy (%) | Occupancy (%) |
| 30 seconds | 0 | 7 |
| 5 minutes | 0 | 29 |
| 10 minutes | 0 | 46 |
| 15 minutes | 0 | 50 |
| 20 minutes | 0 | 54 |

According to Table 3, it can be seen that in the test device in Example 1, blood cells are not leaked at all even after passage of 20 minutes, while in the test device in Comparative Example 1, blood cells have already been leaked after passage of 30 seconds and have reached the judgment part 9 after passage of 15 minutes, to color the judgment part 9, thus making the judgment of examination result difficult. It was thereby demonstrated that according to the test device of the invention in the Example, the chromatographic carrier 7 is not eroded with blood cells in blood even after passage of time after examination.

### 5-2. Comparison between the test device in Example 2 and the test device in Comparative Example 2

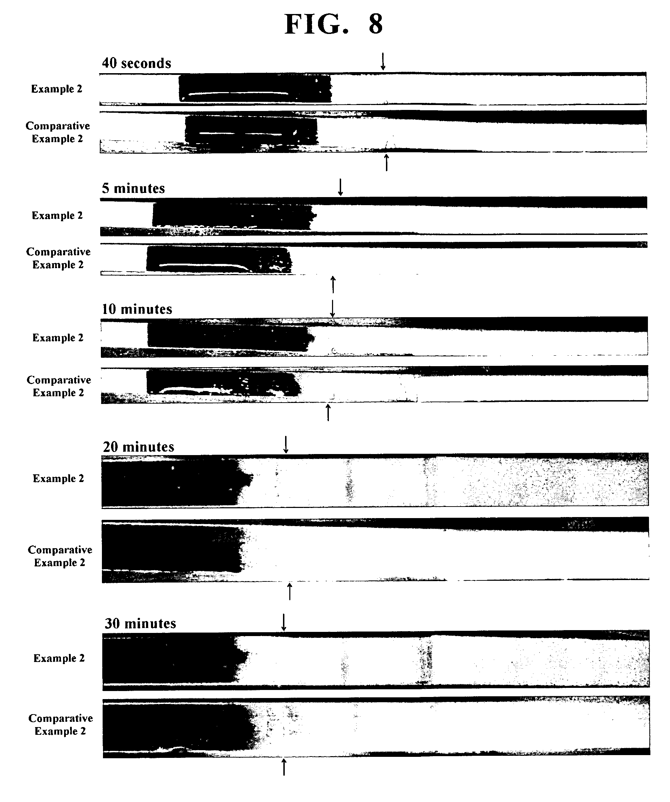

The length of leakage with time of blood cells was observed and measured in the case where 100 µL of whole blood was added to, and developed in, each of the test device in Example 2 and the test device in Comparative Example 2. The results are shown in Table 4 and FIG. 8. FIG. 8 is a photograph showing the developed states of the sample with time in the test devices in Example 2 and Comparative Example 2. In the photograph in FIG. 8, the end, on the downstream side of the direction of sample development, of the label holding member 11 is shown by an arrow. The length of a part on the right side relative to this arrow was regarded as the length of leakage of blood cells. The ratio of the length of leakage of blood cells to the length of that part of the chromatographic carrier 7 that overlaps with neither the label holding member 11 nor the absorbent member 13 was regarded as occupancy and shown in Table 4.

**Table 4**

| | Test device in Example 2 | Test device in Comparative Example 2 |
|---|---|---|
| Elapsed time | Occupancy (%) | Occupancy (%) |
| 40 seconds | 0 | 0 |
| 5 minutes | 0 | 0 |
| 10 minutes | 0 | 0 |
| 25 minutes | 0 | 0 |
| 30 minutes | 0 | 23 |

According to Table 4, it can be seen that in the test device in Example 2, blood cells are not leaked at all even after passage of 30 minutes, while in the test device in Comparative Example 2, blood cells have been leaked after passage of 30 minutes. The test device in Example 2 and the test device in Comparative Example 2, as compared with the test device in Example 1 and the test device in Comparative Example 1, have a lower development speed of the sample, but even in this case, it was demonstrated that according to the test device of the invention in the Example, the chromatographic carrier 7 is not eroded with blood cells in blood even after passage of time after examination.

The foregoing detailed description and examples have been provided by way of explanation and illustration, and are not intended to limit the scope of the appended claims. Many variations in the presentlypreferred embodiments will be obvious to one of ordinary skill in the art, and remain within the scope of the appended claims and their equivalents.

## Claims

1. A chromatographic test device for detecting an analyte in a sample containing red blood cells and liquid components, comprising:
a first blood cell separation member for separating the red blood cells and the liquid components from each other by developing the liquid components more rapidly than the red blood cells;
a second blood cell separation member for separating the red blood cells and the liquid components from each other by capturing the red blood cells and allowing the liquid components to pass; and
a chromatographic carrier carrying a capturing substance being capable of binding specifically to the analyte;
wherein the first blood cell separation member, the second blood cell separation member and the chromatographic carrier are disposed in an order of the first blood cell separationmember, the second blood cell separation member and the chromatographic carrier from an upstream side of the direction of sample development.

2. The chromatographic test device according to claim 1, wherein:
the firstbloodcell separationmember separates the sample in the direction of sample development, and
the second blood cell separation member separates the sample in a direction perpendicular to the direction of sample development.

3. The chromatographic test device according to claim 2, further comprises a sealing member disposed between the first blood cell separation member and the second blood cell separation member,
wherein the sealing member overlaps on a part of the second blood cell separation member, and prevents infiltration of the sample from the first blood cell separation member to the part of the second blood cell separation member

4. The chromatographic test device according to any one of claims 1 to 3, wherein the second blood cell separation member includes a porous member, and the size of pores in the porous member is decreased in the direction of from the upper to lower surfaces of the porous member.

5. The chromatographic test device according to any one of claims 1 to 4, wherein the first blood cell separation member has a sample addition part in its region not overlapping with the second blood cell separation member.

6. The chromatographic test device according to any one of claims 1 to 5, wherein:
at least a part of the first blood cell separation member is disposed so as to overlap on the second blood cell separation member, and
at least a part of the second blood cell separation member is disposed so as to overlap on the chromatographic member.

7. The chromatographic test device according to claim 6, wherein the second blood cell separation member has, on the downstream side of the direction of sample development, a region not being overlapped by the first blood cell separation member.

8. The chromatographic test device according to any one of claims 1 to 7, further comprises a label holding member having a labeling substance being capable of binding specifically to the analyte,
wherein the label holding member is disposed between the second blood cell separation member and the chromatographic carrier, and at least a part of the second blood cell separation member overlaps on the chromatographic member via the label holding member.

9. The chromatographic test device according to claim 8, wherein at least a part of the label holding member is disposed so as to overlap on the chromatographic carrier.

10. The chromatographic test device according to claim 8 or 9, wherein the labeling substance is labeled with colored particles.

11. The chromatographic test device according to any one of claims 1 to 10, further comprises an absorbent member for absorbing the liquid components discharged from the chromatographic carrier,
wherein the first blood cell separation member, the second blood cell separation member, the chromatographic carrier and the absorbent member are disposed in an order of the first blood cell separation member, the second blood cell separation member, the chromatographic carrier and the absorbent member from an upstream side of the direction of sample development.

12. The chromatographic test device according to claim 11, wherein at least a part of the chromatographic carrier is disposed so as to overlap on the absorbent member.

13. The chromatographic test device according to any one of claims 1 to 12, wherein the chromatographic carrier carries the capturing substance on a judgment part.

14. A chromatographic test device for detecting an analyte in a sample containing red blood cells and liquid components, comprising:
a first blood cell separation member including a porous member having pores which are equal to or larger than the red blood cells in size;
a second blood cell separation member having pores smaller than the red blood cells in size; and
a chromatographic carrier carrying a capturing substance being capable of binding specifically to the analyte;
wherein the first blood cell separation member, the second blood cell separation member and the chromatographic carrier are disposed in an order of the first blood cell separationmember, the second blood cell separation member and the chromatographic carrier from an upstream side of the direction of sample development.

15. The chromatographic test device according to claim 14, wherein the first blood cell separation member and the second blood cell separation member are membranous.

16. The chromatographic test device according to claim 14 or 15, wherein the second blood cell separation member is a filtration membrane.

17. The chromatographic test device according to any one of claims 14 to 16, wherein the pores in the second blood cell separation member are decreased in the direction of from the upper to lower surfaces thereof.

18. The chromatographic test device according to any one of claims 14 to 17, further comprises:
a label holding member having a labeling substance being capable of binding specifically to the analyte,
wherein the label holding member is disposed between the second blood cell separation member and the chromatographic carrier.

19. A method for detecting an analyte in a sample containing red blood cells and liquid components by using a chromatographic test device, comprising steps of:
developing the liquid components more rapidly than the red blood cells;
filtrating the red blood cells in the sample; and
detecting the analyte by capturing the analyte on a judgment part carrying a capturing substance being capable of binding specifically to the analyte.

20. The method according to claim 19, which further comprises, after the filtrating step, a step of forming a complex of the analyte and a labeling substance being capable of binding specifically to the analyte.
